# EUROPEAN PATENT APPLICATION

(11) **EP 4 563 694 A1**
(43) Date of publication of application: **04.06.2025**
(21) Application number: 23846450.7
(22) Date of filing: 24.07.2023
(51) Int. Cl.: C12N 1/00, C12N 1/15, C12N 1/19, C12N 1/21, C12N 5/071, C12N 5/10, C12P 21/02, C12P 21/08

(54) **CELL CULTURE MEDIUM**

(30) Priority: 25.07.2022 JP 2022118189
(71) Applicant: AJINOMOTO CO., INC., Chuo-ku Tokyo 104-8315 (JP); CHUGAI SEIYAKU KABUSHIKI KAISHA, Tokyo 115-8543 (JP)
(72) Inventor: MATSUBA, Ayana, Kawasaki-shi, Kanagawa 210-8681 (JP); TSUJI, Chihiro, Kawasaki-shi, Kanagawa 210-8681 (JP); HARADA, Masashi, Kawasaki-shi, Kanagawa 210-8681 (JP); HIGUCHI, Takuya, Kawasaki-shi, Kanagawa 210-8681 (JP); TAKAHASHI, Takeshi, Tokyo 115-8543 (JP)
(74) Representative: Hoffmann Eitle
(86) International application number: PCT/JP2023/026987
(87) International publication number: WO 2024/024720

(57) **Abstract**

Provided are a method, an agent, or the like for improving the stability of a medium and for improving the productivity of a recombinant protein. Specifically, provided are: a medium containing N-acetylcysteine and L-cysteine; a method for culturing a cell using a medium containing N-acetylcysteine and L-cysteine; a method for producing a recombinant protein using a medium containing N-acetylcysteine and L-cysteine; and a method, agent, or the like for stabilizing a medium containing L-cysteine using N-acetylcysteine.

## Description

### TECHNICAL FIELD

The present invention relates to media. Moreover, the present invention relates to methods for culturing cells in media or methods for producing recombinant proteins. Furthermore, the present invention relates to methods or agents for improving the stability of media.

### BACKGROUND ART

Media used in cell culture are often physically or chemically unstable, components may change during preparation, during storage, and/or during cell culture, and this may have a negative impact on the cell culture process. Accordingly, in order to improve productivity and to reduce production costs, particularly when cells are cultured at industrial scales, it is important to develop media having high stability.
The development of highly stable media is important, particularly in terms of improving the productivity of products using recombinant proteins. For example, in the case of antibody drugs using recombinant proteins, the use of highly stable media will enable a more stable and less costly production of antibody drugs for which there is a high demand by society. Moreover, it will also be possible to more effectively perform the research and development of antibody drugs, which requires a tremendous amount of time and costs.

Many attempts to improve media have been reported. For example, JP 2000-507812 A describes stable medium supplementing components that are useful for media that support the growth of hematopoietic cells in culture.

Patent Document 1: JP 2000-507812 A

### SUMMARY OF INVENTION

The present invention can provide media h high stability.

That is, according to the present invention, the embodiments shown below are provided.
(1) A feed medium containing 10-40 mM L-cysteine and 1-40 mM N-acetylcysteine.
(2) A feed medium containing L-cysteine and N-acetylcysteine, wherein a ratio of molar concentrations of N-acetylcysteine to L-cysteine is 0.04-1.0.
(3) The feed medium of (1) or (2), containing 15-30 mM L-cysteine.
(4) The feed medium of any of (1) to (3), containing 2-10 mM N-acetylcysteine.
(5) The feed medium of any of (1) to (4), wherein a ratio of molar concentrations of N-acetylcysteine to L-cysteine is 0.04-1.0.
(6) The feed medium of any of (1) to (5), wherein a ratio of molar concentrations of N-acetylcysteine to L-cysteine is 0.1-0.4.
(7) The feed medium of any of (1) to (6), having a pH of 5.0-9.0.
(8) The feed medium of any of (1) to (7), having a pH of 6.5-7.6.
(9) The feed medium of any of (1) to (8), wherein the feed medium is a serum-free medium and/or an animal protein-free medium.
(10) The feed medium of any of (1) to (9), wherein the feed medium is a synthetic medium.
(11) A powder medium containing L-cysteine and N-acetylcysteine, for preparing a feed medium containing 10-40 mM L-cysteine and 1-40 mM N-acetylcysteine.
(12) A powder medium containing L-cysteine and N-acetylcysteine, for preparing a feed medium having a ratio of molar concentrations of N-acetylcysteine to L-cysteine is 0.04-1.0.
(13) The powder medium of (11) or (12), wherein the feed medium contains 15-30 mM L-cysteine.
(14) The powder medium of any of (11) to (13), wherein the feed medium contains 2-10 mM N-acetylcysteine.
(15) The powder medium of any of (11) to (14), wherein a ratio of molar concentrations of N-acetylcysteine to L-cysteine in the feed medium is 0.04-1.0.
(16) The powder medium of any of (11) to (15), wherein a ratio of molar concentrations of N-acetylcysteine to L-cysteine in the feed medium is 0.1-0.4.
(17) The powder medium of any of (11) to (16), wherein the feed medium has a pH of 5.0-9.0.
(18) The powder medium of any of (11) to (17), wherein the feed medium has a pH of 6.5-7.6.
(19) The powder medium of any of (11) to (18), wherein the feed medium is a serum-free medium and/or an animal protein-free medium.
(20) The powder medium of any of (11) to (19), wherein the feed medium is a synthetic medium.
(21) A method for culturing a cell, including adding the feed medium of any of (1) to (10) to a medium for culturing the cell.
(22) The method of (21), wherein the cell is an animal cell, a plant cell, a bacterial cell, or a fungal cell.
(23) The method of (21) or (22), wherein the cell is an animal cell.
(24) The method of any of (21) to (23), wherein the cell is a mammalian cell.
(25) The method of any of (21) to (24), wherein the cell is a Chinese hamster ovary (CHO) cell.
(26) The method of any of (21) to (25), wherein the cell expresses a recombinant protein.
(27) The method of any of (21) to (26), wherein the recombinant protein includes an antibody or an antigen-binding fragment thereof.
(28) The method of (27), wherein the antibody or the antigen-binding fragment thereof is derived from an IgG.
(29) The method of any of (21) to (28), wherein heat load-induced precipitation of a medium component is absent.
(30) A method for producing a recombinant protein, including adding the feed medium of any of (1) to (10) to a medium for culturing a cell having a capability to produce a recombinant protein.
(31) The method of (30), wherein the cell is an animal cell, a plant cell, a bacterial cell, or a fungal cell.
(32) The method of (30) or (31), wherein the cell is an animal cell.
(33) The method of any of (30) to (32), wherein the cell is a mammalian cell.
(34) The method of any of (30) to (33), wherein the cell is a Chinese hamster ovary (CHO) cell.
(35) The method of any of (30) to (34), wherein the recombinant protein includes an antibody or an antigen-binding fragment thereof.
(36) The method of (35), wherein the antibody or the antigen-binding fragment thereof is derived from an IgG.
(37) The method of any of (30) to (36), wherein heat load-induced precipitation of a medium component is absent.
(38) A method for improving stability of a liquid medium, including adding L-cysteine and/or N-acetylcysteine to a medium to achieve an L-cysteine concentration of 10-40 mM and an N-acetylcysteine concentration of 1-40 mM.
(39) A method for improving stability of a liquid medium, including adding L-cysteine and/or N-acetylcysteine to a medium to achieve a ratio of molar concentrations of N-acetylcysteine to L-cysteine of 0.04-1.0.
(40) A method for suppressing generation and/or precipitation of cystine in a liquid medium, including adding L-cysteine and/or N-acetylcysteine to a medium to achieve an L-cysteine concentration of 10-40 mM and an N-acetylcysteine concentration of 1-40 mM.
(41) A method for suppressing generation and/or precipitation of cystine in a liquid medium, including adding L-cysteine and/or N-acetylcysteine to a medium to achieve a ratio of molar concentrations of N-acetylcysteine to L-cysteine of 0.04-1.0.
(42) The method of any of (38) to (41), including adding L-cysteine and N-acetylcysteine to the medium.
(43) The method of any of (38) to (41), wherein the liquid medium contains 10-40 mM L-cysteine, and the method includes adding N-acetylcysteine to the medium to achieve an N-acetylcysteine concentration of 1-40 mM.
(44) The method of any of (38) to (41), wherein the liquid medium contains 1-40 mM N-acetylcysteine, and the method includes adding L-cysteine to the medium to achieve an L-cysteine concentration of 10-40 mM.
(45) The method of any of (38) to (44), including adding L-cysteine and/or N-acetylcysteine to the medium to achieve an L-cysteine concentration of 15-30 mM.
(46) The method of any of (38) to (45), including adding L-cysteine and/or N-acetylcysteine to the medium to achieve an N-acetylcysteine concentration of 2-10 mM.
(47) The method of any of (38) to (46), including adding L-cysteine and/or N-acetylcysteine to the medium to achieve a ratio of molar concentrations of N-acetylcysteine to L-cysteine of 0.04-1.0.
(48) The method of any of (38) to (47), including adding L-cysteine and/or N-acetylcysteine to the medium to achieve a ratio of molar concentrations of N-acetylcysteine to L-cysteine of 0.1-0.4.
(49) The method of any of (38) to (48), including adding L-cysteine and/or N-acetylcysteine to the medium to achieve a pH of 5.0-9.0 for the liquid medium.
(50) The method of any of (38) to (49), including adding L-cysteine and/or N-acetylcysteine to the medium to achieve a pH of 6.5-7.6 for the liquid medium.
(51) An agent containing L-cysteine and N-acetylcysteine, for improving stability of a liquid medium, wherein the liquid medium, after addition of the agent, contains 10-40 mM L-cysteine and 1-40 mM N-acetylcysteine.
(52) An agent containing L-cysteine and N-acetylcysteine, for improving stability of a liquid medium, wherein a ratio of molar concentrations of N-acetylcysteine to L-cysteine in the liquid medium, after addition of the agent, is 0.04-1.0.
(53) An agent containing N-acetylcysteine, for improving stability of a liquid medium containing 10-40 mM L-cysteine, wherein the liquid medium, after addition of the agent, contains 1-40 mM N-acetylcysteine.
(54) An agent containing N-acetylcysteine, for improving stability of a liquid medium containing L-cysteine, wherein a ratio of molar concentrations of N-acetylcysteine to L-cysteine in the liquid medium, after addition of the agent, is 0.04-1.0.
(55) An agent containing L-cysteine and N-acetylcysteine, for suppressing generation and/or precipitation of cystine in a liquid medium, wherein the liquid medium, after addition of the agent, contains 10-40 mM L-cysteine and 1-40 mM N-acetylcysteine.
(56) An agent containing L-cysteine and N-acetylcysteine, for suppressing generation and/or precipitation of cystine in a liquid medium, wherein a ratio of molar concentrations of N-acetylcysteine to L-cysteine in the liquid medium, after addition of the agent, is 0.04-1.0.
(57) An agent containing N-acetylcysteine, for suppressing generation and/or precipitation of cystine in a liquid medium containing 10-40 mM L-cysteine, wherein the liquid medium, after addition of the agent, contains 1-40 mM N-acetylcysteine.
(58) An agent containing N-acetylcysteine, for suppressing generation and/or precipitation of cystine in a liquid medium containing L-cysteine, wherein a ratio of molar concentrations of N-acetylcysteine to L-cysteine in the liquid medium, after addition of the agent, is 0.04-1.0.
(59) The agent of any of (51) to (58), wherein the liquid medium, after addition of the agent, contains 15-30 mM L-cysteine.
(60) The agent of any of (51) to (59), wherein the liquid medium, after addition of the agent, contains 2-10 mM N-acetylcysteine.
(61) The agent of any of (51) to (60), wherein a ratio of molar concentrations of N-acetylcysteine to L-cysteine in the liquid medium, after addition of the agent, is 0.04-1.0.
(62) The agent of any of (51) to (61), wherein a ratio of molar concentrations of N-acetylcysteine to L-cysteine in the liquid medium, after addition of the agent, is 0.1-0.4.
(63) The agent of any of (51) to (62), wherein the liquid medium, after addition of the agent, has a pH of 5.0-9.0.
(64) The agent of any of (51) to (63), wherein the liquid medium, after addition of the agent, has a pH of 6.5-7.6.
(65) A media additive containing N-acetylcysteine and L-cysteine, for preparing the feed medium of any of (1) to (10) or the powder medium of any of (11) to (20).
(66) A media additive containing N-acetylcysteine, for preparing the feed medium of any of (1) to (10) or the powder medium of any of (11) to (20).

The present invention can improve the stability of media.

### BRIEF DESCRIPTION OF DRAWINGS

Figure 1 provides graphs that show changes over time in concentrations of L-cysteine, cystine, N-acetylcysteine, N-acetylcystine, and N,N'-diacetylcystine in liquid media.
Figure 2 provides photographs that show the effect of N-acetylcysteine on the precipitation of medium components in media containing L-cysteine.
Figure 3 provides a graph that shows the effect of N-acetylcysteine on viable cell densities in media containing L-cysteine. Viable cell densities on days 9 and 14 from the start of culture are shown.
Figure 4 provides a graph that shows the effect of N-acetylcysteine on antibody yields in media containing L-cysteine. Antibody yields on days 7, 9, 11, and 14 from the start of culture are shown.
Figure 5 provides a graph that shows the effect of L-cysteine concentration on antibody yields in media. Relative values of antibody yields on day 14 from the start of culture are shown.

### DESCRIPTION OF EMBODIMENTS

### (Description of Terms)

The terms herein, unless otherwise specified, have the meanings indicated below.

A "cell culture medium" in the present disclosure refers to a medium that provides a growing environment for cells, which is used for growing or maintaining cells. A cell culture medium typically contains components that are essential and components that are useful for cell growth and survival.

A "feed medium" in the present disclosure refers to a component that is added, after a cell culture process has started, by supplementing a medium that is being used in the cell culture.
A feed medium typically contains components that are essential and components that are useful in terms of achieving cell growth and survival. The feed medium may be a composition containing only one of these components and may be a composition containing a plurality of the components. For example, in the present disclosure, liquids containing only L-cysteine and/or N-acetylcysteine are also included in feed media. The feed medium may contain a component(s) (e.g., a sugar (glucose), amino acid, or the like) of a cell culture medium. The expression "medium" may be used to mean a cell culture medium and may be used to mean a feed medium. The term includes both meanings.

A feed medium is typically used in cell culture using a fed-batch culturing method. The fed-batch culturing method is a culturing method in which at the start of the culturing process, a basal medium containing all of the components (including cells and all nutrient sources) for cell culture is supplied to a culture vessel, and then a feed medium is added to the culture vessel continuously or in stages. Here, the basal medium is a medium that is used from the start of the cell culturing process, and so long as the medium is one that can be used as a basal medium, it can be used as the basal medium without particular limitations.
Feed media are generally provided or sold in the form of liquid media or powder media. The storage of feed media typically uses plastic bottles and stainless tanks but is not limited thereto.

There are no particular limitations as to the timing at which the feed medium is added to the culture vessel. For example, the addition may take place at the start of the cell culture, in the lag phase, in the logarithmic growth phase, in the stationary phase, and/or in the death phase. Moreover, the feed medium may be used in other culturing methods, e.g., a continuous culturing method. The continuous culturing method, also known as a perfusion culturing method, is a culturing method in which an additional medium (such as a feed medium) is supplied to the culture vessel at a constant speed and at the same time, the same amount of a culture fluid is removed from the culture vessel.

Non-limiting examples of components that are essential and components that are useful for cell growth and survival include, e.g., carbohydrates such as sugars (e.g., glucose), lipids, nucleic acids, vitamins (e.g., thiamine), essential and non-essential amino acids, ammonium salts, nitrate salts, minerals (e.g., phosphorus, sulfur, potassium, magnesium, iron, and salts thereof), trace metals (e.g., cobalt, copper, zinc, nickel, and salts thereof), sera and substitutes thereof, animal proteins, animal-derived components (e.g., hydrolysates), plant proteins, plant-derived components (e.g., hydrolysates), microbial proteins, microorganism-derived components (e.g., hydrolysates), trophic/growth factors, hormones, enzyme cofactors, pH buffers, sodium chloride, calcium chloride, antibiotics, preservatives (e.g., antioxidants), and pH indicators (e.g., phenol red).

Non-limiting examples of essential and non-essential amino acids include alanine, cysteine, aspartic acid, glutamic acid, phenylalanine, glycine, histidine, isoleucine, lysine, leucine, methionine, asparagine, proline, glutamine, arginine, serine, threonine, valine, tryptophan, tyrosine, cystine, hydroxyproline, thyroxine, phosphoserine, desmosine, β-alanine, sarcosine, ornithine, citrulline, and γ-aminoglactic acid, as well as salts thereof and hydrates thereof,
The concentrations of essential and non-essential amino acids in a medium are selected, as appropriate, according to the purpose of the culture and the cell. When the medium is a liquid medium and is used as a feed medium, the total concentration of essential and non-essential amino acids contained in the medium is typically 10-1000 mM.
Non-limiting examples of pH buffers include sodium carbonate buffers, potassium carbonate buffers, sodium phosphate buffers, phosphate buffers, trishydroxymethylaminomethane buffers, citrate buffers, and Good's buffers (e.g., HEPES buffers and MES buffers). When the medium contains a pH buffer, the concentration of the pH buffer is typically 10-500 mM.

According to the cell to be cultured and the purpose, the medium can be selected, as appropriate, from commercially available media and known media described in literature. According to the purpose and cell of the culture, the constitution may be changed, an agent or a media additive may be added to the medium, or two or more media may be mixed and used. Moreover, an additional medium, agent, and/or media additive may be added to the medium during cell culture.
The medium may be a synthetic medium. Moreover, the medium may be a serum-free medium, and may be an animal protein-free medium. The medium may be a liquid medium or a powder medium.

Non-limiting examples of commercially available media include Dulbecco's Modified Eagle Medium (DMEM), DME/F12, Eagle's Minimum Essential Medium (MEM), Basal Medium Eagle (BME), F-10 medium, F-12 medium, α-Minimum Essential Medium (α-MEM), Glasgow Minimum Essential Medium (G-MEM), PF CHO Medium, Iscove's Modified Dulbecco's Medium, AmpliCHO CD medium, Dynamis^{™} Medium, EX-CELL^{™} Advanced^{™} CHO Fed-Batch Medium, CD FortiCHO^{™} Medium, CP OptiCHO^{™}, BalanCD^{™} CHO Growth A Medium, ActiPro^{™}, Roswell Park Memorial Institute 1640 (RPMI-1640) medium, CELList^{™} Basal Media (BASAL 3, BASAL 10), and Feed Media (FEED 2), HyClone^{™} cell Boost^{™} 7a Supplement, Cellvento^{™} Feed-200, Cellvento^{™} Feed-210, Cellvento^{™} Feed-220, as well as Cellvento^{™} 4CHO COMP BalanCD CHO Feed 4. These media can be used as cell culture media and can also be used as feed media. Moreover, these media are provided or sold in the forms of liquid media and/or powder media.

A "synthetic medium" in the present disclosure refers to a medium prepared using only chemically-defined components. A synthetic medium is typically a medium in which sera and substitutes thereof, unpurified animal proteins, unpurified animal-derived components (e.g., hydrolysates), unpurified plant proteins, unpurified plant-derived components (e.g., hydrolysates), unpurified microbial proteins, and unpurified microorganism-derived components (e.g., hydrolysates) have not been added.

A "serum-free medium" in the present disclosure refers to a medium free of unadjusted or unpurified serum. Media contaminated with purified blood-derived components or animal tissue-derived components (e.g., growth factors), so long as they do not contain unadjusted or unpurified sera, are also included in the serum-free medium.

An "animal protein-free medium" in the present disclosure refers to a medium that does not contain animal proteins (e.g., animal-derived proteins such as albumin, transferrin, insulin, and growth factors). Animal protein-free media may contain small peptides and/or oligopeptides.

A "powder medium" in the present disclosure refers to a medium in powder form. Powder media also include media in granular form. Moreover, powder media also include dried media. A powder medium can be made into a liquid medium by dissolving the powder medium in an appropriate solvent (e.g., water). Any of the media in the present disclosure can be prepared or provided in the form of a powder medium.

A "liquid medium" in the present disclosure refers to a medium in a liquid form. Any of the media in the present disclosure can be prepared or provided in the form of a liquid medium. Moreover, a liquid medium can be prepared by dissolving any of the powder media in the present disclosure in an appropriate solvent (e.g., water). The liquid medium can be used as a feed medium.

A "recombinant protein" in the present disclosure refers to a polymer of amino acids of an arbitrary length made using an arbitrary genetic recombination technique. The polymer may be linear or branched, may contain a modified amino acid, and may contain a component other than amino acids. Moreover, the recombinant protein may undergo a chemical or biological modification, such as disulfide bond formation, glycosylation, lipidation, acetylation, phosphorylation, amidation, derivatization by a protecting group or the like, cleavage, or conjugation with a labeling component (e.g., a radioisotope) or a drug (e.g., a toxin, a cytotoxic drug, or a radioisotope). Molecules including a recombinant protein moiety as part of the structure thereof are all included in recombinant proteins in the present disclosure. The genetic recombination technique is not particularly limited so long as it is a technique that artificially engineers genes, but typical examples thereof include techniques that introduce a nucleic acid encoding a recombinant protein into a host cell.

Non-limiting examples of recombinant proteins include antibodies and antigen-binding fragments thereof, antibody mimetics, immunoadhesins, enzymes, hormones, cytokines, clotting factors (or blood clotting-associated proteins), extracellular proteins, and membrane proteins produced using genetic recombination techniques. These recombinant proteins are mainly used in fields of medicine, agrochemicals, food, and other chemical industries, but are not limited to a specific use.

An "antibody" in the present disclosure refers to an immunoglobulin molecule with antigen binding properties. Antibodies include full-length antibodies, which have polypeptide chains including a light chain and a heavy chain, and fragments thereof (e.g., Fv, Fab, Fab', F(ab')₂, and VHH fragments). An antibody may include a heavy chain and/or a light chain. The heavy chain and/or the light chain may include a variable region (involved in antigen recognition and binding) and a constant region (involved in localization and intracellular interactions). Most common full-length antibodies include two heavy chain constant regions (CHs), two heavy chain variable regions (VHs), two light chain constant regions (CLs), and two light chain variable regions (VLs). The variable region includes complementarity-determining regions (CDRs), which are sequences that impart antigen specificity to the antibody, and framework regions (FRs).
Antibodies include monoclonal antibodies, multispecific antibodies (e.g., bispecific antibodies) formed from two or more antibodies or antigen binding sites, antibody fragments with desired biological activities, and fusion proteins including the antigen-binding sites of antibodies. Moreover, antibodies include chimeric antibodies, mammalian antibodies (e.g., human antibodies), mammalianized antibodies (e.g., humanized antibodies), multivalent antibodies, and modified antibodies.
An antibody may be of any class (e.g., IgG, IgA, IgM, IgD, IgE) or of any subclass (isotype).
An antibody may undergo a biological or chemical modification, such as conjugation with another molecule (e.g., a toxin of low molecular weight or high molecular weight (cytotoxic drug), or a radioisotope), and antibodies having undergone these modifications are also included in the antibodies according to the present invention.

An "antibody mimetic" in the present disclosure refers to a protein that can specifically bind to an antigen but is not structurally related to an antibody. A specific example of an antibody mimetic is the Z domain (Affibody) of protein A, and a more specific example is a ZHER2 affibody.

Examples of enzymes include alkaline phosphatase, β-lactamase, galactosidase, glucosidase, glucocerebrosidase, superoxide dismutase, and DNase.

Examples of hormones include renin, growth hormone (human growth hormone, bovine growth hormone, or the like), parathyroid hormone, thyroid stimulating hormone, calcitonin, gonadotropins (e.g., follicle-stimulating hormone and leutinizing hormone), glucagon, insulin (insulin A chain and insulin B chain), and proinsulin.

Examples of cytokines include tumor necrosis factors (e.g., TNF-α and - β), vascular endothelial growth factor (VEGF), brain-derived neurotrophic factor (BDNF), neurotrophins (e.g., NT-3 to NT-6), nerve growth factor (NGF), platelet-derived growth factor (PDGF), fibroblast growth factors (e.g., aFGF and bFGF), epidermal growth factor (EGF), transforming growth factors (e.g., TGF-α and TGF-β1 to TGF-β5), activins (e.g., activin A, activin C, and activin E), insulin-like growth factors (e.g., IGF-I and IGF-II), hepatocyte growth factor (HGF), keratinocyte growth factor, stem cell factor (SCF), bone morphogenetic proteins (BMP), RANTES, erythropoietin (EPO), thrombopoietin (TPO), interferons-α, -β, and -γ, colony-stimulating factors (e.g.. M-CSF, GM-CSF, and G-CSF), and interleukins (e.g., IL-1 to IL-13).

Examples of clotting factors and blood clotting-associated proteins include factor VII, factor VIII, factor VIIIC, factor IX, factor X, factor XI, factor XII, tissue factor, von Willebrand factor, protein C, protein S, plasminogen activators (e.g., urokinase, human urine, or human tissue plasminogen activators (t-PA)), thrombin, prothrombin, thrombopoietin, thrombomodulin, antithrombin III, fibrinogen, and serum albumin.

Examples of extracellular proteins include fibronectin, vitronectin, collagen, osteopontin, laminin, and fragments thereof.

Examples of membrane proteins include membrane receptor proteins, membrane transport proteins, membrane enzymes, and cell adhesion factors, as well as fragments thereof.

The recombinant protein is not limited by the examples mentioned above. For example, receptors of the above-mentioned cytokines or the like, transferrin, various CD proteins, tumor-associated antigens (e.g., HER2, HER3, or HER4 receptor, CA125), viral antigens (e.g., viral envelope proteins) for vaccines, and the like are also included in the recombinant protein. Moreover, fusion proteins including any of the proteins mentioned above are also included in the recombinant protein.

The expression "having a capability to produce a recombinant protein" in the present disclosure means being capable of producing a protein by a genetic recombination technique. The produced recombinant protein may be secreted extracellularly (i.e., into the medium) or may accumulate in the cell, but is preferably secreted extracellularly.

Typically, an ability to produce a recombinant protein can be imparted to a host cell by introducing into the host cell a nucleic acid encoding the recombinant protein. That is, a typical cell having a capability to produce a recombinant protein is a cell containing a nucleic acid encoding the recombinant protein. Regarding the method for introducing a nucleic acid into a host cell, various methods are known in the relevant technical field. In the present disclosure, these various methods are included, and any method publicly known at the time the present invention is implemented can be used. A non-limiting example of such a method is a method for introducing a vector containing a nucleic acid encoding a recombinant protein into a host cell. The vector is preferably an expression vector containing a nucleic acid encoding a recombinant protein in an expressible form (e.g., in a form operably linked to a regulatory sequence such as a promoter, an enhancer, a ribosome binding sequence and/or a transcription termination sequence) and may be a plasmid.

Non-limiting examples of methods for introducing a vector into a host cell include methods in which a vector is physically or chemically introduced into a host cell by electroporation, particle gun, calcium phosphate, or lipofection, and methods in which a host cell is infected with a viral vector. Moreover, the nucleic acid in the vector introduced into a host cell may be incorporated into the genome of the host cell by a genome editing technique such as CRISPR/Cas9. Alternatively, instead of introducing a nucleic acid encoding a recombinant protein into a host cell, a gene sequence on genomic DNA may be directly modified by a genome editing technique such as CRISPR/Cas9 to encode a recombinant protein of interest, or a control sequence or the like may be modified to control (e.g., improve) the production of an endogenous protein of interest. All proteins produced in such a manner are also included in the recombinant protein in the present disclosure.

The term "cell" in the present disclosure includes animal cells, plant cells, bacterial cells, and fungal cells. The cell is preferably an established cell line that can be suitably used in the production of a recombinant protein.
The animal cell may be a cell derived from any animal, but is preferably a cell derived from a mammal (*Mammalia*), a bird, an amphibian, a fish, or an insect, more preferably a cell derived from a mammal or an insect, and most preferably a cell derived from a mammal.

Non-limiting examples of cells derived from mammals include cells derived from primates such as humans or monkeys and cells derived from rodents such as mice, rats, or hamsters.
Non-limiting examples of cells derived from mammals include cell lines such as HL-60 (ATCC No. CCL-240), HT-1080 (ATCC No. CCL-121), HeLa (ATCC No. CCL-2), 293 (ECACC No. 85120602), Hep G2 (ATCC No. HB8065), VERO (ATCC No. CCL-1651), CV1 (ATCC No. CCL70), COS-7 (ATCC No. CRL-1651), NIH3T3 (ATCC No. CRL-1658), NS0 (ATCC No. CRL-1827), Chinese hamster ovary (CHO) cells, BHK21 (also referred to simply as BHK cells; ATCC No. CRL-10), and MDCK (ATCC No. CCL-34), and sublines derived from these cell lines (e.g., BHK TK- cells, which are a subline of BHK cells, and CHO-K1 cells, CHO-S cells, CHO-DXB11 cells (also referred to as CHO-DUKX cells or DuxB11 cells), and CHO-DG44 cells, which are sublines of CHO cells).

A non-limiting example of a cell derived from a bird is the chicken cell line SL-29. A non-limiting example of a cell derived from an amphibian is a Xenopus oocyte. A non-limiting example of a cell derived from a fish is the zebrafish cell line ZF4.

A non-limiting example of a cell derived from an insect is a cell derived from Spodoptera frugiperda. Non-limiting examples of cells derived from Spodoptera frugiperda include Sf9 cells, Sf21 cells, and SF+ cells.

There are no particular limitations to the plant cell so long as the cell is a collected cell and/or tissue that can be cultured in liquid media. Non-limiting examples of plant cells include plants (e.g., medicinal ginseng, periwinkle, henbane, *Coptisjaponica,* or *Atropa belladonna*) from which crude drugs (e.g., saponin, alkaloids, berberine, scopolin, or plant sterols) are produced, plants (e.g., blueberries, safflower, *Rubia tinctorum,* or saffron) from which pigments and polysaccharides (e.g., anthocyanin, safflower pigments, madder pigments, saffron pigments, or flavones) that serve as raw materials for cosmetics/food are produced, or plants from which pharmaceutical raw materials are produced.

The bacterial cell is a eubacterial (Gram-positive or Gram-negative) or archaeal cell. Non-limiting examples of bacterial cells include *Escherichia* bacterial cells (e.g., *Escherichia coli*), *Bacillus* bacterial cells (e.g., *Bacillus subtilis*), *Pseudomonas* bacterial cells (e.g., *Pseudomonas fluorescens*), *Corynebacterium* bacterial cells, and *Lactococcus* bacterial cells (e.g., *Lactococcus lactis*).,

Fungal cells are large cells that have organelles and a classic nucleus and belong to *Eukaryota.* Non-limiting examples of fungal cells include *Saccharomyces* fungi (e.g., *Saccharomyces cerevisiae*), *Schizosaccharomyces* fungi (e.g., *Schizosaccharomyces pombe*), and *Pichia* fungi (e.g., *Pichia pastoris*).

The "L-cysteine" in the present disclosure includes L-cysteine salts that function as L-cysteine in solutions (e.g., solutions with water, a buffer, or a medium as the solvent), hydrates of L-cysteine, and hydrates of L-cysteine salts. However, in the context of an L-cysteine concentration, the concentration means the concentration of L-cysteine that functions in a solution.
The L-cysteine concentration in a solution is typically represented by the L-cysteine concentration at the time the solution is prepared (a calculated value is preferred, but a measured value taken immediately after preparation may also be used). However, in the context of a change in concentration over time after a solution is prepared, the L-cysteine concentration means the L-cysteine concentration at the point when a predetermined amount of time has passed after the solution is prepared.
Non-limiting examples of L-cysteine salts include L-cysteine hydrochloride, L-cysteine sodium salt, and L-cysteine ammonium salt. Non-limiting examples of L-cysteine hydrates include L-cysteine hydrochloride monohydrate, L-cysteine sodium salt monohydrate, and L-cysteine ammonium salt monohydrate. As the L-cysteine, it is also possible to use a combination of a plurality of substances from among L-cysteine, L-cysteine salts, and hydrates thereof.

The "N-acetylcysteine" in the present disclosure includes N-acetylcysteine salts that function as N-acetylcysteine in solutions (e.g., solutions with water, a buffer, or a medium as the solvent), hydrates of N-acetylcysteine, and hydrates of N-acetylcysteine salts. However, in the context of an N-acetylcysteine concentration, the concentration means the concentration of N-acetylcysteine that functions in a solution.
N-acetylcysteine may be in the L-form and may be in the D-form. It may also be a mixture of L- and D-forms.
The N-acetylcysteine concentration in a solution is typically represented by the N-acetylcysteine concentration at the time the solution is prepared (a calculated value is preferred, but a measured value taken immediately after preparation may also be used). However, in the context of a change in concentration over time after a solution is prepared, the N-acetylcysteine concentration means the N-acetylcysteine concentration at the point when a predetermined amount of time has passed after the solution is prepared.
Non-limiting examples of N-acetylcysteine salts include N-acetylcysteine sodium salt and N-acetylcysteine ammonium salt. Non-limiting examples of N-acetylcysteine hydrates include N-acetylcysteine sodium salt monohydrate and N-acetylcysteine ammonium salt monohydrate. As the N-acetylcysteine, it is also possible to use a combination of a plurality of substances from among N-acetylcysteine, N-acetylcysteine salts, and hydrates thereof.

The "heat load" in the present disclosure, when applied to a medium during preparation, during storage, and/or during cell culture, means applying heat at a temperature that can adversely affect the medium. Non-limiting examples of such adverse effects include precipitation of medium components in the medium during preparation, storage, and/or cell culture, as well as reduced quantity and/or quality of a recombinant protein produced in the medium. The temperature that can adversely affect the medium may be a temperature (typically 4-40 °C) media can usually take at the time of preparation, storage, and/or cell culture. It may also be a temperature that includes higher temperatures (e.g., 4-120 °C).

The "stability" in the present disclosure, when used in connection with media, refers to a property of physical and chemical changes not occurring or rarely occurring during preparation, during storage, and/or during cell culture. For example, when the generation rate of a byproduct (e.g., cystine generated from L-cysteine) in a medium is slow, when there is no precipitation of a medium component (e.g., cystine), or when the time (number of days or the like) to precipitation is long, the stability of the medium can be said to be high.

A "media additive" in the present disclosure refers to any substance or composition containing the substance that is added to a medium. The media additive may be for supplementing a component already existing in the medium to which the addition is made or for newly supplementing a component that is not present in the medium to which the addition is made. Media additives are typically provided in liquid form or powder form (including granular form). Moreover, the purpose of the addition is not particularly limited.

The singular forms "a", "an", and "the" used in the present disclosure, unless clearly indicated otherwise, can include plural subjects.
The term "and/or" used in the present disclosure includes both the relationship represented by "and" and the relationship represented by "or". The terms "also" and "as well as" may also be used to express "and". The term "otherwise" may also be used to express "or". The term "comprising/containing/including" as used in the present disclosure includes "consisting mainly of", "consisting substantially of" and "consisting of". The term "consisting mainly of" includes "consisting substantially of" and "consisting of", and "consisting substantially of" includes "consisting of".

Each numerical range in the present disclosure includes the upper limit and the lower limit indicated by "-" or "to". For example, a description of "A-B" or "A to B" using numerical values A and B means A or more and B or less. Moreover, a description of "A-B", "A to B" or "A or more and B or less" in numerical ranges described in stages in the present disclosure independently includes both "A or more is preferred" and "B or less is preferred", and these lower limits or upper limits may be replaced by the upper limits or lower limits in other numerical ranges. Moreover, the lower limit or upper limit of a numerical range described in the present disclosure may also be replaced with a numerical value that is within the numerical range and is shown in an Example.

### (Embodiments)

Herebelow, the present invention will be explained in detail using embodiments for carrying out the invention, but the present invention is not limited thereby.
These embodiments may be carried out alone or as a combination of a plurality thereof. For the definitions and details in the embodiments, reference can be made to the "Description of Terms" above.
Known techniques and procedures used in the present disclosure are sufficiently understood by those skilled in the art and can be performed in accordance with conventional methods.

In one embodiment, a feed medium (or a liquid medium) containing L-cysteine and N-acetylcysteine is provided. In another embodiment, a powder medium containing N-acetylcysteine and L-cysteine for preparing a feed medium (or a liquid medium) is provided. Moreover, in another embodiment, a method for preparing a feed medium (or a liquid medium) or a powder medium is provided. Further, in another embodiment, a media additive for preparing a feed medium (or a liquid medium) or a powder medium is provided. In these embodiments, the liquid medium is preferably a feed medium.
In the liquid media (hereinafter including feed media in the form of liquid media) of these embodiments,
(1) the L-cysteine concentration is 1-40 mM, preferably 10-40 mM, 10-30 mM, or 15-40 mM, and more preferably 15-30 mM, and/or the N-acetylcysteine concentration is 1-40 mM, preferably 1-30 mM, 1-20 mM, or 1-10 mM, and more preferably 2-10 mM; and/or
(2) the ratio of molar concentrations of N-acetylcysteine to L-cysteine is 0.04-2.0, preferably 0.04-1.0, 0.04-0.4, 0.1-2.0, 0.1-1.0, or 0.1-0.4, and more preferably 0.2-0.4.

The liquid medium in these embodiments, by containing N-acetylcysteine at a suitable concentration or content ratio, preferably has suppressed formation and/or precipitation of cystine from L-cysteine and/or has improved stability. Moreover, the liquid medium in these embodiments preferably contains a high concentration of L-cysteine that is advantageous for the growth or proliferation of the cultured cell. By using such a liquid medium, a cell culture process can be stably performed. Moreover, preferably, costs associated with cell culture are reduced. Further, by using such a liquid medium in the production of a recombinant protein, it is possible to improve the productivity of a product using the recombinant protein. Preferably, the production costs of products using recombinant proteins are reduced. Preferably, the research and development of products using recombinant proteins are made more efficient.

The liquid medium in these embodiments is not limited to a particular constitution so long as it contains L-cysteine and N-acetylcysteine, and any liquid medium can be used. The pH of the liquid medium may be 5.0-9.0, and is preferably 6.0-8.0, more preferably 6.5-7.5, and most preferably 6.5-7.0. The liquid medium may be a serum-free medium and/or animal protein-free medium. Moreover, the liquid medium may be a synthetic medium. The liquid medium may be used in a large-scale process (e.g., cell culture at a 500 L-scale).

When the liquid medium in these embodiments is used in culturing a cell having a capability to produce a recombinant protein, the productivity of the recombinant protein can be improved. That is, when the various conditions associated with cell culture are optimized, typically, it is possible to produce a recombinant protein at 1-20 g/L, preferably 2-16 g/L, more preferably 6-14 g/L, even more preferably 8-12 g/L, and most preferably 10-12 g/L. Moreover, there is preferably no precipitation of a component in the liquid medium due to heat load during preparation, during storage, and/or during cell culture.

The powder medium in these embodiments contains L-cysteine and N-acetylcysteine. The powder medium is not limited to a particular constitution so long as it contains L-cysteine and N-acetylcysteine, and any powder medium can be used. The powder medium can be used to prepare a liquid medium of an embodiment of the present disclosure by dissolving the powder medium in a solvent (e.g., water). In this situation, the powder medium contains L-cysteine and N-acetylcysteine at amounts for achieving desired concentrations of L-cysteine and N-acetylcysteine in a liquid medium. The powder medium may be accompanied by an instruction, an explanation on a website, or the like, for diluting the concentrations of L-cysteine and N-acetylcysteine in a liquid medium to desired concentrations.
Compared with liquid media, powder media are generally easy to handle and are advantageous in terms of, e.g., more stable storage and distribution.

The method for preparing a liquid medium or a powder medium includes mixing L-cysteine, N-acetylcysteine, components that are essential and components that are useful for cell growth and survival, and the like to obtain a liquid medium or powder medium of any of the embodiments of the present disclosure. For L-cysteine and/or N-acetylcysteine, the media additive or agent of any of the embodiments of the present disclosure can also be used. According to the method, those skilled in the art can prepare a liquid medium or a powder medium as appropriate.

The media additive contains L-cysteine and/or N-acetylcysteine. The media additive may be added to either a cell culture medium or a feed medium, or the media additive may be added to both media. The media additive is not limited to a particular constitution so long as it contains L-cysteine and/or N-acetylcysteine, and any constitution can be used. The media additive may contain only L-cysteine and/or N-acetylcysteine, or it may be a composition containing a carrier, a pH adjuster, a medium, or the like. The media additive may be in the form of a liquid. It may also be in the form of a solid, e.g., the form of a powder (including the form of granules).

The media additive can be added to any liquid medium. In this situation, the media additive typically contains L-cysteine and/or N-acetylcysteine at amounts for achieving desired concentrations of L-cysteine and N-acetylcysteine in a liquid medium. Moreover, the media additive can be added to any powder medium. In this situation, the media additive typically contains L-cysteine and/or N-acetylcysteine at amounts for achieving desired concentrations of L-cysteine and N-acetylcysteine in a liquid medium when the liquid medium is prepared by dissolving a powder medium, to which the media additive has been added, in a solvent (e.g., water).
Moreover, the media additive may be accompanied by an instruction, an explanation on a website, or the like, for diluting the concentrations of L-cysteine and N-acetylcysteine in a liquid medium to desired concentrations.

By adding the media additive, a liquid medium or a powder medium of an embodiment of the present disclosure can be obtained.
The media additive is for use in the preparation of a liquid medium or a powder medium. The use is preferably described in a sales brochure, a user's manual, or the like for the media additive. In this situation, the use can be described as an effect of the media additive.

In one embodiment, a method for culturing a cell, including adding a liquid medium of an embodiment of the present disclosure to a medium for culturing the cell is provided. Moreover, in another embodiment, a method for producing a recombinant protein, including adding a liquid medium of an embodiment of the present disclosure to a medium for culturing a cell having a capability to produce a recombinant protein is provided.
A medium for culturing a cell is typically a medium that has been placed in a culture vessel during cell culture. Moreover, a medium for culturing a cell is typically a basal medium or a basal medium to which a feed medium has been added. As the medium for culturing a cell and the feed medium, a liquid medium (including a liquid medium prepared from a powder medium or a liquid medium to which a media additive has been added) of any of the embodiments of the present disclosure can be used for each. A liquid medium that is added to a medium for culturing a cell is typically a feed medium.

By using a medium with improved stability and/or a medium with suppressed formation and/or precipitation of cystine, the method for culturing a cell preferably enables stable performance of cell culture. Moreover, the method for culturing a cell preferably reduces costs associated with culturing.

By using a medium with improved stability and/or a medium with suppressed formation and/or precipitation of cystine, the method for producing a recombinant protein enables an improvement in the productivity of a product using the recombinant protein. Moreover, the method for producing a recombinant protein preferably reduces production costs of products using the recombinant protein. Moreover, the method for producing a recombinant protein preferably makes a contribution to increased efficiency in the research and development of products using the recombinant protein.

The cell may be an animal cell, a plant cell, a bacterial cell, or a fungal cell, and may preferably be an animal cell, more preferably a mammalian cell, and even more preferably a Chinese hamster ovary (CHO) cell. Moreover, the cell may express a protein. The protein is typically a recombinant protein. The recombinant protein may include an antibody or an antigen-binding fragment thereof. The antibody or the antigen-binding fragment thereof may be derived from an IgG.

The number of times of adding the liquid medium to the medium for culturing the cell is not particularly limited. The addition may be performed once or a plurality of times. There are also no particular limitations with regard to when the liquid medium is added to the medium for culturing a cell. The addition may be performed at any one of the stages or at a plurality of the stages of: before culturing is started, when culturing is started, and after culturing has been started. Moreover, the liquid medium may be added to the cell being cultured continuously or in stages. Moreover, the liquid medium may be added to the medium for culturing the cell alone or together with another medium, agent, or media additive. Moreover, when the liquid medium is added, the same amount of the culture fluid may be removed at the same time.
The amount of the liquid medium added to the medium for culturing the cell is typically 1-20% and preferably 2-10% of the amount of the medium for culturing the cell. The L-cysteine concentration in the medium for culturing the cell after addition of the liquid medium is typically 0.01-8 mM and preferably 0.02-4 mM. The N-acetylcysteine concentration in the medium for culturing the cell after addition of the liquid medium is typically 0.01-8 mM and preferably 0.02-4 mM.
The method for culturing a cell and the method for producing a recombinant protein may be used in large-scale processes (e.g., cell culture at a 500 L-scale).

The method for culturing a cell may include preparing a medium. Moreover, the method for culturing a cell may include culturing the cell. The method for culturing a cell typically follows a fed-batch culturing method, but may also follow another culturing method (e.g., continuous culturing method).

The method for producing a recombinant protein may include preparing a medium. Moreover, the method for producing a recombinant protein may include culturing a cell. Moreover, the method for producing a recombinant protein may include producing the recombinant protein. Moreover, the method for producing a recombinant protein may include collecting a produced recombinant protein. Moreover, the method for producing a recombinant protein may include other additional steps. Non-limiting examples of additional steps include purifying a recombinant protein and chemically or biologically modifying the recombinant protein. Usually, a plurality of methods is used in combination in purifying a recombinant protein, and chromatography can be suitably used. In particular, when the recombinant protein is an antibody, affinity chromatography, protein A chromatography, and the like can be suitably used.

Non-limiting examples of chemical or biological modifications on recombinant proteins include, e.g., disulfide bond formation, glycosylation, lipidation, acetylation, phosphorylation, amidation, derivation by a protecting group or the like, cleavage, or conjugation with a labeling component or a drug (a toxin or the like). The method for producing a recombinant protein also includes a method for producing an antibody conjugated with a drug such as a toxin. In this situation, the method for producing a recombinant protein may further include, as an additional step, conjugating an antibody and a drug. An antibody conjugated with a drug is included in the recombinant protein of the present disclosure.

According to the method for producing a recombinant protein of the present disclosure, it is possible to produce a recombinant protein at a high yield. That is, according to the method for producing a recombinant protein of the present disclosure, when conditions associated with cell culture are optimized, typically, it is possible to produce a recombinant protein at 1-20 g/L, preferably 2-16 g/L, more preferably 6-14 g/L, even more preferably 8-12 g/L, and most preferably 10-12 g/L. Moreover, according to the method for producing a recombinant protein of the present disclosure, there is preferably no precipitation of a medium component in the liquid medium due to heat load during preparation, during storage, and/or during cell culture.

In one embodiment, a method or agent for improving stability of a liquid medium (or a feed medium), including adding L-cysteine and/or N-acetylcysteine to the medium, is provided. In another embodiment, a method or agent for suppressing the generation and/or precipitation of cystine in a liquid medium (or a feed medium), including adding L-cysteine and/or N-acetylcysteine to a medium, is provided. In another embodiment, use of L-cysteine and/or N-acetylcysteine for improving the stability of a liquid medium (or a feed medium) is provided. In another embodiment, use of L-cysteine and/or N-acetylcysteine for suppressing the generation and/or precipitation of cystine in a liquid medium (or a feed medium) is provided. The liquid medium in these embodiments is preferably a feed medium.

In these embodiments, L-cysteine and/or N-acetylcysteine are added to the liquid medium such that:
(1) the L-cysteine concentration is 1-40 mM, preferably 10-40 mM, 10-30 mM, or 15-40 mM, and more preferably 15-30 mM, and/or the N-acetylcysteine concentration is 1-40 mM, preferably 1-30 mM, 1-20 mM, or 1-10 mM, and more preferably 2-10 mM; and/or
(2) the ratio of molar concentrations of N-acetylcysteine to L-cysteine is 0.04-2.0, preferably 0.04-1.0, 0.04-0.4, 0.1-2.0, 0.1-1.0, or 0.1-0.4, and more preferably 0.2-0.4,
or the concentrations or ratio in the liquid medium, after addition of the above-mentioned agent, are within the above-mentioned ranges.

According to the method, agent, or use of these embodiments, by containing N-acetylcysteine at a suitable concentration or content ratio, it is possible to improve the stability of a liquid medium containing L-cysteine and/or suppress the formation and/or precipitation of cystine in the liquid medium. Therefore, according to the method, agent, or use of these embodiments, it is possible to stably perform cell culture at industrial scales.
Moreover, when the method, agent, or use of these embodiments is used in a process for producing a recombinant protein, the productivity of a product using the recombinant protein can be improved. Preferably, the production costs of products using recombinant proteins are reduced. Preferably, the research and development of products using recombinant proteins are more efficient.

The method, agent, or use of these embodiments can improve the stability of a liquid medium containing L-cysteine and N-acetylcysteine compared with a liquid medium that contains L-cysteine but is free of N-acetylcysteine. The improvement in the stability of a liquid medium may be evaluated by a known method, or may simply be for the purpose of improving stability without evaluation. Non-limiting examples of methods for evaluating stability include methods in which precipitation of a medium component (e.g., cystine) is detected visually or by spectroscopic means (e.g., by a turbidity meter) and stability is evaluated based on the time (number of the days or the like) taken to generate the precipitation, and methods in which stability is evaluated by analyzing a change in the concentration of a medium component (e.g., cystine) using physicochemical analysis means. Non-limiting examples of physicochemical analysis means include, e.g., means using a liquid chromatograph-tandem mass spectrometer as described in WO 2021/060517 A.

In a further embodiment of the present disclosure, the method or agent for improving the stability of a liquid medium lengthens the time (number of days or the like) taken to generate precipitation in a liquid medium containing L-cysteine and N-acetylcysteine, as compared with a liquid medium that contains L-cysteine but is free of N-acetylcysteine. The lengthened time is, for example, one day or more, preferably five days or more, more preferably 10 days or more, and even more preferably 15 days or more. Moreover, in another embodiment, the method, agent, or use for suppressing the generation and/or precipitation of cystine in a liquid medium lowers the generation speed of a byproduct (e.g., cystine) in a liquid medium containing L-cysteine and N-acetylcysteine by 10% or more, preferably 20% or more, more preferably 30% or more, and even more preferably 40% or more, as compared with a liquid medium that contains L-cysteine but is free of N-acetylcysteine. Moreover, in yet another embodiment, the method, agent, or use for suppressing the generation and/or precipitation of cystine in a liquid medium keeps a byproduct (e.g., cystine) from precipitating in a liquid medium containing L-cysteine and N-acetylcysteine for a period of two weeks or more, preferably three weeks or more, more preferably one month or more, even more preferably two months or more, and most preferably three months or more.

The method, agent, or use for suppressing the generation and/or precipitation of cystine in a liquid medium can suppress the generation and/or precipitation of cystine in a liquid medium containing L-cysteine and N-acetylcysteine as compared with a liquid medium that contains L-cysteine but is free of N-acetylcysteine. The generation and/or precipitation of cystine in a liquid medium may be evaluated by a known method or may simply be for the purpose of suppressing the generation and/or precipitation of cystine in a liquid medium without evaluation. Non-limiting examples of methods for evaluating the suppression of the generation and/or precipitation of cystine include methods in which the precipitation of cystine is detected visually or by spectroscopic means (e.g., by a turbidity meter) and the generation and/or precipitation of cystine is evaluated based on the time (number of the days or the like) taken to generate the precipitation, and methods in which the generation and/or precipitation of cystine is evaluated by analyzing a change in the concentration of cystine using physicochemical analysis means. Non-limiting examples of physicochemical analysis means include, e.g., means using a liquid chromatograph-tandem mass spectrometer as described in WO 2021/060517 A.

In a further embodiment of the present disclosure, the method, agent, or use for suppressing the generation and/or precipitation of cystine in a liquid medium lengthens the number of days for cystine to precipitate in a liquid medium containing L-cysteine and N-acetylcysteine, as compared with a liquid medium that contains L-cysteine but is free of N-acetylcysteine. The lengthened time is, for example, one day or more, preferably five days or more, more preferably 10 days or more, and even more preferably 15 days or more. Moreover, in another embodiment, the method, agent, or use for suppressing the generation and/or precipitation of cystine in a liquid medium lowers the generation and/or precipitation speed of cystine in a liquid medium containing L-cysteine and N-acetylcysteine by 10% or more, preferably 20% or more, more preferably 30% or more, and even more preferably 40% or more, as compared with a liquid medium that contains L-cysteine but is free of N-acetylcysteine.

The pH of the liquid medium after L-cysteine and/or N-acetylcysteine have been added to the medium may be 5.0-9.0, and is preferably 6.0-8.0, more preferably 6.5-7.5, and most preferably 6.5-7.0. The characteristics of the liquid medium after L-cysteine and/or N-acetylcysteine have been added to the medium may be identical to the characteristics of any one of the liquid media described in the present disclosure.

Adding L-cysteine and/or N-acetylcysteine to a liquid medium includes all of the following: (1) adding L-cysteine and/or N-acetylcysteine or a composition containing the same to a liquid medium; (2) mixing L-cysteine and/or N-acetylcysteine or a composition containing the same with another component or another composition (a medium or the like) containing another component, and then adding the mixture to a medium; and (3) mixing N-acetylcysteine or a composition containing the same with another component or another composition (a medium or the like) containing another component to make a medium.

A liquid medium to which L-cysteine and/or N-acetylcysteine or the above agent is added, prior to the addition, may contain L-cysteine and/or N-acetylcysteine. In this situation, the desired concentrations of L-cysteine and N-acetylcysteine in the liquid medium may be achieved after L-cysteine and/or N-acetylcysteine or the agent has been added.

The above agent is not particularly limited to a particular form so long as it contains L-cysteine and/or N-acetylcysteine, and the agent may contain only L-cysteine and/or N-acetylcysteine, or may be in the form of a composition containing a carrier, a pH adjuster, a medium or the like. Moreover, the agent may be in the form of a liquid. It may also be in the form of a solid, e.g., the form of a powder (including the form of granules).
The above agent is preferably for use in the improvement of the stability of a liquid medium or in the suppression of the generation and/or precipitation of cystine in a liquid medium. The use is preferably described in a sales brochure for the agent, a user's manual, a website for the product, or the like. In this situation, the use can be described as an effect of the agent.

Additionally, the media, methods, agents, additives, uses, and the like described using the above embodiments and the like do not limit the present invention. They are disclosed under the intention of illustration. The technical scope of the present invention is as defined by the recitations of the claims, and those skilled in the art can make various design changes within the technical scope of the invention as claimed.

For example, in the above description of embodiments, explanations have been made focusing on feed media, but the embodiments of the present disclosure are not limited to the feed media and can be applied to any liquid media including those not used in feeds. That is, another embodiment of the present disclosure is any of the embodiments above in which the feed medium is replaced by a liquid medium (including one that is not a feed medium).

For example, in one embodiment, a recombinant protein produced by an embodiment of the present disclosure is provided. Moreover, another embodiment relates to a pharmaceutical composition or medicament containing the recombinant protein, use of the recombinant protein in the manufacture of a medicament, or a method for treating a disease including administering the recombinant protein. Moreover, other embodiments relate to methods for producing a pharmaceutical composition or pharmaceutical formulation, including any of the steps according to the embodiments of the present disclosure or any of the methods according to the embodiments of the present disclosure, including obtaining a recombinant protein thereby, and further including mixing the recombinant protein and a carrier.

### EXAMPLES

Herebelow, the present invention will be explained using examples, but these examples do not limit the present invention. The commercially available reagents, machines, etc. mentioned in the examples, unless specifically indicated, were used according to the manufacturer's use instructions or conventional methods.

### (Example 1)

### Effects of N-Acetylcysteine on Liquid Stability of Feed Media Having High L-Cysteine Concentrations

Feed Media A were prepared on the basis of the commercially available CELLiST^{™} Feed media (AJINOMOTO CO., INC.: FEED 2). Feed Media A do not contain N-acetylcysteine.
N-acetylcysteine at final concentrations of 0-5.5 mM was added to Feed Media A, which contained 60 g/L glucose and L-cysteine at final concentrations of 18-26 mM to prepare liquid media of pH 6.7-6.9 according to the procedures described in the product instructions for FEED 2. The prepared liquid media were filtered in 200 mL portions using 150 mL capacity Stericup-GP (Merck: S2GPU01RE) and stored for 18 days at 4 °C or 25 °C, protected from light.

The results of visually detecting precipitations after storage are shown in Table 1. In the Feed Medium A in which N-acetylcysteine was not added, precipitation was detected after 18 days. Meanwhile, in the Feed Media A in which 18 mM L-cysteine was added, precipitation was not detected when 2.7-5.5 mM N-acetylcysteine was further added.
Moreover, in the Feed Media A in which 22 mM L-cysteine was added, precipitation was not detected when 2.7-5.5 mM N-acetylcysteine was further added.
Further, in Feed Media A in which 24 mM or 26 mM L-cysteine was added, precipitation was detected when 3.7 mM N-acetylcysteine was added. Meanwhile, precipitation was not detected when 5.5 mM N-acetylcysteine was added.

**Table 1**

| Medium | L-cysteine concentration (mM) | N-acetylcysteine added concentration (mM) | 4°C 18 days | 25°C 18 days |
|---|---|---|---|---|
| Feed Medium A | 18 | 0 | Precipitation | Precipitation |
| Feed Medium A | 18 | 2.7 | No precipitation | No precipitation |
| Feed Medium A | 18 | 3.7 | No precipitation | No precipitation |
| Feed Medium A | 18 | 5.5 | No precipitation | No precipitation |
| Feed Medium A | 22 | 2.7 | No precipitation | No precipitation |
| Feed Medium A | 22 | 3.7 | No precipitation | No precipitation |
| Feed Medium A | 22 | 5.5 | No precipitation | No precipitation |
| Feed Medium A | 24 | 3.7 | Precipitation | Precipitation |
| Feed Medium A | 24 | 5.5 | No precipitation | No precipitation |
| Feed Medium A | 26 | 3.7 | Precipitation | Precipitation |
| Feed Medium A | 26 | 5.5 | No precipitation | No precipitation |

The results above showed that the addition of N-acetylcysteine suppresses the generation of precipitates in liquid media containing L-cysteine at high concentrations and improves the stability of the liquid media, as compared with the case in which N-acetylcysteine was not added.

### (Example 2)

### Effects of N-acetylcysteine on Liquid Stability of Media Having High L-Cysteine Concentrations and Cystine Concentrations During Media Storage

Feed Media A were prepared on the basis of the commercially available CELLiST^{™} Feed media (AJINOMOTO CO., INC.: FEED 2). Feed Media A do not contain N-acetylcysteine.
N-acetylcysteine at final concentrations of 0-5.5 mM was added to Feed Media A, in which final concentrations of 18 or 24 mM L-cysteine and 60 g/L glucose were added, to prepare liquid media of pH 6.7-6.9 according to the procedures described in the product instructions for FEED 2. The prepared liquid media were filtered using Stericup-GP, and 25 mL or 50 mL portions were placed in 60 mL capacity PETG square media bottles (Thermo Fisher Scientific: 2019-0030) and stored for 21 days at 4 °C, protected from light.

The results of visually detecting the precipitation of crystals after storage are shown in Table 2. In the Feed Media A containing 18 mM L-cysteine, precipitation was detected after 8 or 13 days when N-acetylcysteine was not added. Meanwhile, precipitation was not detected when 5.5 mM N-acetylcysteine was added.
Moreover, in the Feed Media A containing 24 mM L-cysteine, precipitation was detected after 7 days when N-acetylcysteine was not added. Meanwhile, precipitation was detected after 8 or 13 days when 2.8 mM N-acetylcysteine was added. Moreover, when 5.5 mM N-acetylcysteine was added, precipitation was detected after 21 days under the 25 mL liquid volume condition. Precipitation was not detected under the 50 mL liquid volume condition.

**Table 2**

| Medium | L-cysteine concentration (mM) | N-acetylcysteine added concentration (mM) | Liquid volume (mL) | Day on which precipitation was first observed |
|---|---|---|---|---|
| Feed Medium A | 18 | 0 | 50 | 8 |
| Feed Medium A | 18 | 0 | 25 | 13 |
| Feed Medium A | 18 | 5.5 | 50 | Not observed |
| Feed Medium A | 18 | 5.5 | 25 | Not observed |
| Feed Medium A | 24 | 0 | 50 | 7 |
| Feed Medium A | 24 | 0 | 25 | 7 |
| Feed Medium A | 24 | 2.8 | 50 | 13 |
| Feed Medium A | 24 | 2.8 | 25 | 8 |
| Feed Medium A | 24 | 5.5 | 50 | Not observed |
| Feed Medium A | 24 | 5.5 | 25 | 21 |

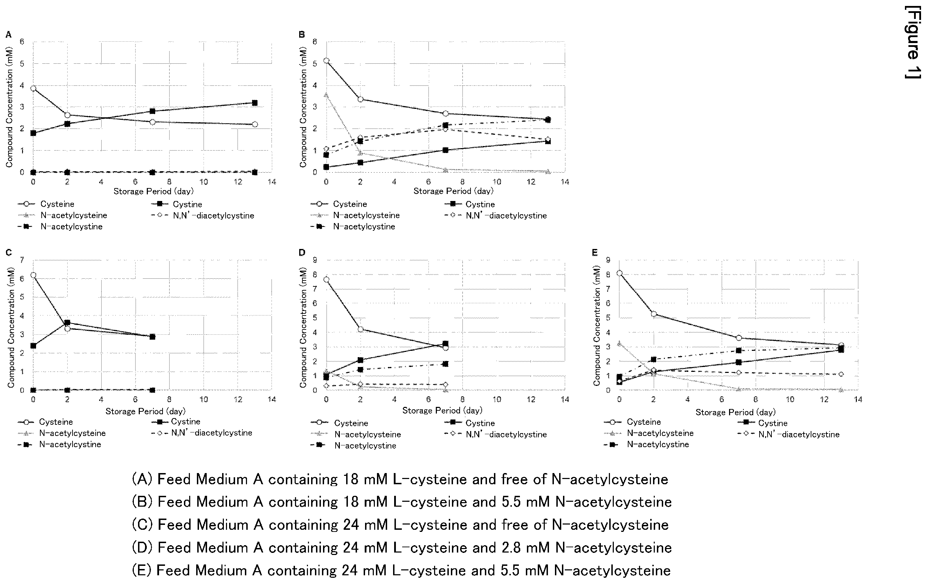

Next, for five media under the 25 mL liquid volume condition used in Table 2, an analysis of changes over time in the concentrations of L-cysteine, cystine, N-acetylcysteine, N-acetylcystine, and N,N'-diacetylcystine in the liquids was performed according to the method described in WO 2021/060517 A.
The results are shown in Figure 1. In the media in which N-acetylcysteine was not added (Figures 1A and 1C), the cystine concentration in the liquids increased and precipitation was detected. Meanwhile, in the media in which N-acetylcysteine was added (Figures 1B, 1D, and 1E), N-acetylcystine was generated. Compared with the media in which N-acetylcysteine was not added, the generation speed of cystine slowed down.

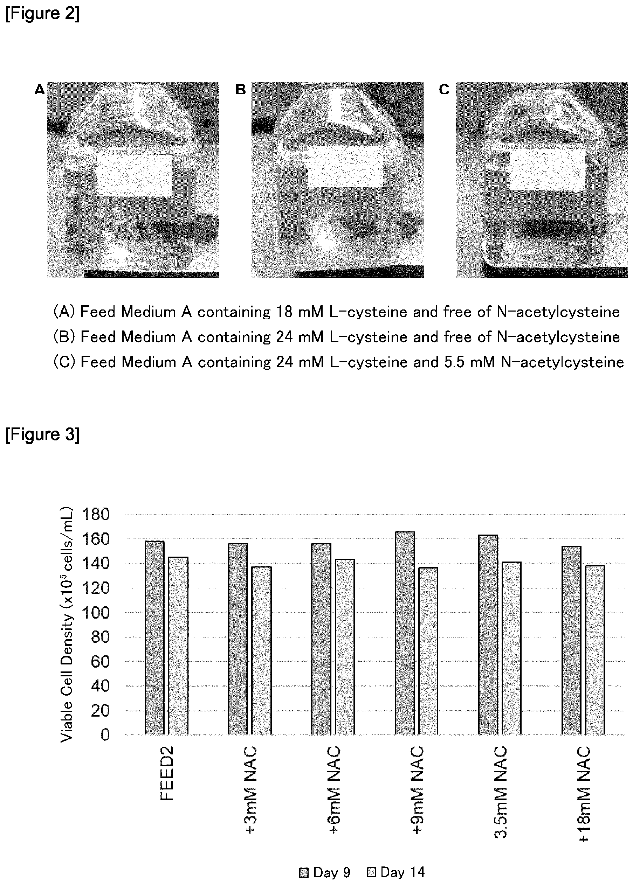

Further, for (A) a liquid medium containing 18 mM L-cysteine and free of N-acetylcysteine, (B) a liquid medium containing 24 mM L-cysteine and free of N-acetylcysteine, and (C) a liquid medium in which 24 mM L-cysteine and 5.5 mM N-acetylcysteine were added (the liquid value of each liquid medium was 50 mL), 10 mL of liquid medium was removed on day 13 after preparation, storage was continued until day 63, and then precipitation was visually detected.
The results are shown in Figure 2. Precipitation was detected in N-acetylcysteine-free liquid media (A) and (B). Meanwhile, precipitation was not detected in N-acetylcysteine-containing liquid medium (C).

The above results showed that the generation of precipitates is suppressed and the generation speed of cystine slows down in liquid media which contain L-cysteine and in which N-acetylcysteine has been added.

### (Example 3)

### Effects of N-Acetylcysteine on Liquid Stability of Media Having High L-Cysteine Concentrations

Commercially available CELLiST^{™} Feed media (AJINOMOTO CO., INC.: FEED 2) were used. FEED 2 does not contain N-acetylcysteine. To FEED 2 that contains 72 g/L glucose, a final concentration of 18 mM L-cysteine and a final concentration of 0, 3, or 9 mM N-acetylcysteine were further added. The prepared liquid media were filtered using Stericup-GP, then 10 or 40 mL portions were placed in 50 mL Falcon tubes and stored at 25 °C, protected from light.

The results of visually detecting the precipitation of crystals after storage are shown in Table 3. In the media in which 18 mM L-cysteine was added to FEED 2, precipitation was detected after 2 days under the 10 mL liquid volume condition and after 5 days under the 40 mL liquid volume condition.
In the media in which 18 mM L-cysteine and 3 mM N-acetylcysteine were added to FEED 2, precipitation was detected after 5 days under the 10 mL liquid volume condition and after 10 days under the 40 mL liquid volume condition.
In the media in which 18 mM L-cysteine and 9 mM N-acetylcysteine were added to FEED 2, precipitation was detected after 10 days under the 10 mL liquid volume condition and after 19 days under the 40 mL liquid volume condition.

**Table 3**

| Medium | L-cysteine added concentration (mM) | N-acetylcysteine added concentration (mM) | Liquid volume (mL) | Day on which precipitation was first observed |
|---|---|---|---|---|
| FEED 2 | 18 | 0 | 10 | 2 |
| FEED 2 | 18 | 0 | 40 | 5 |
| FEED 2 | 18 | 3 | 10 | 5 |
| FEED 2 | 18 | 3 | 40 | 10 |
| FEED 2 | 18 | 9 | 10 | 10 |
| FEED 2 | 18 | 9 | 40 | 19 |

The above results showed that the addition of N-acetylcysteine suppresses the generation of precipitates in media with high L-cysteine concentrations and improves liquid stability.

### (Example 4)

### Effects of N-Acetylcysteine on Liquid Stability of Feed Media

Commercially available CELLiST^{™} Feed media (AJINOMOTO CO., INC.: FEED 2) were used. FEED 2 does not contain N-acetylcysteine. To FEED 2 that contains 72 g/L glucose, 3, 6, 9, 13.5, or 18 mM N-acetylcysteine was added. The prepared liquid media were filtered using Stericup-GP, and then under conditions promoting oxidation reaction, 10 mL portions were placed in 50 mL Falcon tubes and stored for 32 days at 25 °C, protected from light.

The results of visually detecting the precipitation of crystals after storage are shown in Table 4. In the FEED 2 medium in which N-acetylcysteine was not added, precipitation was detected after 22 days. Meanwhile, in the media in which 3, 6, 9, 13.5, or 18 mM N-acetylcysteine was added to FEED 2, precipitation was not detected.

**Table 4**

| Medium | N-acetylcysteine added concentration (mM) | Day on which precipitation was first observed |
|---|---|---|
| FEED 2 | 0 | 22 |
| FEED 2 | 3 | Not observed |
| FEED 2 | 6 | Not observed |
| FEED 2 | 9 | Not observed |
| FEED 2 | 13.5 | Not observed |
| FEED 2 | 18 | Not observed |

The above results showed that the addition of N-acetylcysteine suppresses the generation of precipitates in media with high L-cysteine concentrations and improves liquid stability.

### (Example 5)

### Effects of N-Acetylcysteine Addition to Feed Media on CHO Cell Growth and Antibody Production

Basal media were prepared by adding LR3-IGF-1 (SIGMA-ALDRICH: I1271-1MG, final concentration 50 µg/mL) and L-Glutamine (Thermo Fisher Scientific: 25030081, final concentration 6 mM) to CELLiST^{™} Basal media (AJINOMOTO CO., INC.: BASAL3).
The six media with different N-acetylcysteine concentrations used in Example 4 were used soon after they were prepared.
CHO cell suspensions, made using the prepared basal media and adjusted to 3 × 10⁵ cells/mL, were seeded in 6-well plates (CORNING, Cat. No. CLS3471). The culture was started at 4 mL and was performed for 14 days. All of the experimental groups were performed with n = 2. The culture temperature was 37 °C and the stirring speed was 110 rpm. On days 4, 7, 9, and 11 after start of the culture, feed media were added at 7% relative to the media in the plates. Cell culture fluids were sampled on days 7, 9, 11, and 14 after start of the culture. Viable cell numbers were measured using the automated cell viability analyzer Vi-CELLTM XR (Beckman Coulter, Inc.), and antibody yields were measured using Octet QK (FORTEBIO).

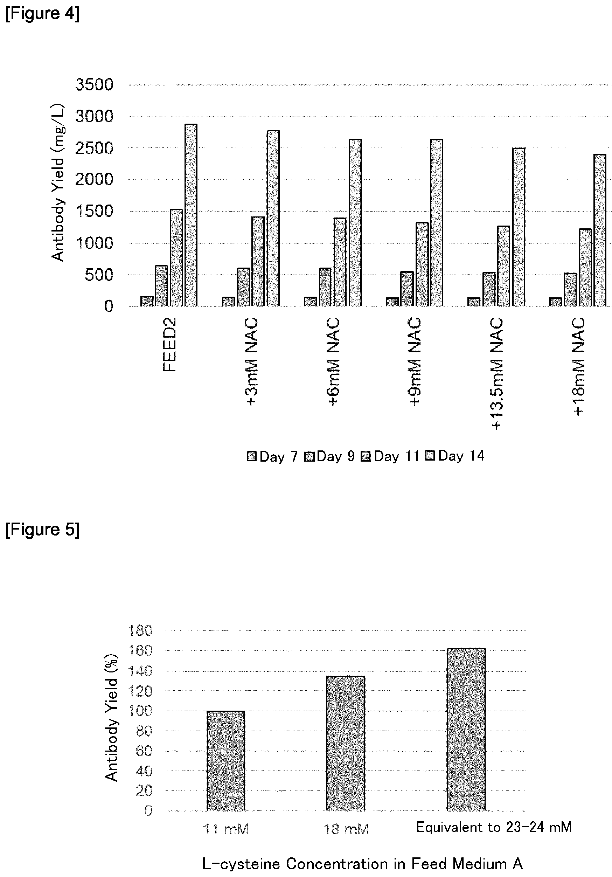

The results are shown in Figures 3 and 4. Even in the media in which 3, 6, 9, 13.5, or 18 mM N-acetylcysteine was added to the feed media, sufficient cell growth and antibody production were achievable.
These results showed that even when N-acetylcysteine is added, superior cell growth ability and recombinant protein productivity, provided by media with high L-cysteine concentrations, are maintained.

### (Example 6)

### Effects of Feed Media Having High L-Cysteine Concentrations on CHO Cell Growth and Antibody Production

Basal media were prepared by adding a final concentration of 4 mM L-Glutamine (Thermo Fisher Scientific: 25030081) to media that were designed on the basis of CELLiST^{™} Basal media (AJINOMOTO CO., INC.: BASAL10).
Feed Media A were prepared on the basis of the commercially available CELLiST^{™} Feed media (AJINOMOTO CO., INC.: FEED 2). Feed media A containing a final concentration of 11 or 18 mM L-cysteine and 60 g/L glucose were prepared to have a pH of 6.7-6.9 according to the procedures described in the product instructions for FEED 2.
A 110 mM L-cysteine solution (pH 11.3) was prepared.
Using the prepared basal media, CHO suspensions adjusted to 7 × 10⁵ cells/mL were cultured for 14 days using the ambr15 cell culture system (Sartorius AG). All of the experimental groups were performed with n = 2. The experimental groups include: conditioned sections (11 mM section and 18 mM section) in which Feed Media A containing 11 or 18 mM L-cysteine were added daily from day 2 after start of the culture at 3.3% each; and a conditioned section (23-24 mM equivalent section) in which Feed Medium A containing 18 mM L-cysteine was added daily from day 2 after start of the culture at 3.3% each and the 110 mM L-cysteine solution was added daily from day 2 after start of the culture at 0.163%. The antibody yields on day 14 in these conditioned sections were measured using Cedex Bio HT (Roche Diagnostics K.K.)

The results are shown in Figure 5. It was shown that an increase in the amount of L-cysteine supplied during culture improves the antibody yield.

### (Example 7)

### Effects of N-Acetylcysteine on Liquid Stability of Media Having High L-Cysteine Concentrations

Commercially available CELLiST^{™} Feed media (AJINOMOTO CO., INC.: FEED 2) were used. FEED 2 does not contain N-acetylcysteine. A final concentration of 0, 4, 9, or 18 mM L-cysteine and a final concentration of 0, 2, 10, or 20 mM N-acetylcysteine were further added to FEED 2. The prepared liquid media were filtered using Stericup-GP, then 10 or 40 mL portions were placed in 50 mL Falcon tubes and stored for 29 days at 4 °C, protected from light.
The results of visually detecting the precipitation of crystals after storage are shown in Table 5.

**Table 5**

| Medium | L-cysteine added concentration (mM) | N-acetylcysteine added concentration (mM) | Liquid volume (mL) | Day on which precipitation was first observed |
|---|---|---|---|---|
| FEED2 | 0 | 0 | 10 | No precipitation |
| FEED2 | 0 | 2 | 10 | No precipitation |
| FEED2 | 0 | 10 | 10 | No precipitation |
| FEED2 | 0 | 20 | 10 | No precipitation |
| FEED2 | 4 | 0 | 10 | 14 |
| FEED2 | 4 | 2 | 10 | 19 |
| FEED2 | 4 | 10 | 10 | No precipitation |
| FEED2 | 4 | 20 | 10 | No precipitation |
| FEED2 | 9 | 0 | 10 | 6 |
| FEED2 | 9 | 2 | 10 | 10 |
| FEED2 | 9 | 10 | 10 | 29 |
| FEED2 | 9 | 20 | 10 | No precipitation |
| FEED2 | 18 | 0 | 10 | 3 |
| FEED2 | 18 | 2 | 10 | 3 |
| FEED2 | 18 | 10 | 10 | 11 |
| FEED2 | 18 | 20 | 10 | 21 |
| FEED2 | 0 | 0 | 40 | No precipitation |
| FEED2 | 0 | 2 | 40 | No precipitation |
| FEED2 | 0 | 10 | 40 | No precipitation |
| FEED2 | 0 | 20 | 40 | No precipitation |
| FEED2 | 4 | 0 | 40 | 19 |
| FEED2 | 4 | 2 | 40 | 19 |
| FEED2 | 4 | 10 | 40 | No precipitation |
| FEED2 | 4 | 20 | 40 | No precipitation |
| FEED2 | 9 | 0 | 40 | 10 |
| FEED2 | 9 | 2 | 40 | 10 |
| FEED2 | 9 | 10 | 40 | 21 |
| FEED2 | 9 | 20 | 40 | No precipitation |
| FEED2 | 18 | 0 | 40 | 3 |
| FEED2 | 18 | 2 | 40 | 3 |
| FEED2 | 18 | 10 | 40 | 11 |
| FEED2 | 18 | 20 | 40 | 16 |

As shown in Table 5, precipitation was not observed after 29 days in the FEED 2 media in which L-cysteine was not added whether under the 10 mL liquid volume condition or the 40 mL liquid volume condition.
In the media in which 4 mM L-cysteine was added to FEED 2, precipitation was detected after 14 days under the 10 mL liquid volume condition and after 19 days under the 40 mL liquid volume condition. Under the 10 mL liquid volume condition, the addition of 2 mM N-acetylcysteine delayed the day on which precipitation was detected to 19 days, and in media in which 10 or 20 mM N-acetylcysteine was added, precipitation was not observed up to 29 days. Under the 40 mL liquid volume condition, precipitation was not observed up to 29 days in the media in which 10 or 20 mM N-acetylcysteine was added.
In the media in which 9 mM L-cysteine was added to FEED 2, precipitation was detected after 6 days under the 10 mL liquid volume condition and after 10 days under the 40 mL liquid volume condition. Under the 10 mL liquid volume condition, the addition of 2 or 10 mM N-acetylcysteine delayed the day on which precipitation was detected to 10 days and 29 days respectively, and in media in which 20 mM N-acetylcysteine was added, precipitation was not observed up to 29 days. Under the 40 mL liquid volume condition, the addition of 10 mM N-acetylcysteine delayed the day on which precipitation was detected to 21 days, and in media in which 20 mM N-acetylcysteine was added, precipitation was not observed up to 29 days.
Moreover, in the media in which 18 mM L-cysteine was added to FEED 2, precipitation was detected after 3 days whether under the 10 mL liquid volume condition or the 40 mL liquid volume condition. Under the 10 mL liquid volume condition, the addition of 10 or 20 mM N-acetylcysteine delayed the day on which precipitation was detected to 11 days and 21 days respectively. Under the 40 mL liquid volume condition, the addition of 10 or 20 mM N-acetylcysteine delayed the day on which precipitation was detected to 11 days and 16 days respectively.

The results above showed that the addition of N-acetylcysteine suppresses the generation of precipitates in liquid media containing L-cysteine at high concentrations and improves the stability of the liquid media, as compared with cases in which N-acetylcysteine is not added.

The disclosures of the patents, patent applications, and publications cited in the present disclosure are incorporated herein by reference in their entirety.

### INDUSTRIAL APPLICABILITY

The media of the present embodiments can improve the productivity of products using recombinant proteins, so the media can be suitably used in, e.g., the production of antibody drugs using recombinant proteins, and have industrial applicability.

## Claims

1. A feed medium comprising 10-40 mM L-cysteine and 1-40 mM N-acetylcysteine.

2. A feed medium comprising L-cysteine and N-acetylcysteine, wherein a ratio of molar concentrations of N-acetylcysteine to L-cysteine is 0.04-1.0.

3. The feed medium of claim 1, comprising 15-30 mM L-cysteine.

4. The feed medium of claim 1, comprising 2-10 mM N-acetylcysteine.

5. The feed medium of claim 1, wherein a ratio of molar concentrations of N-acetylcysteine to L-cysteine is 0.04-1.0.

6. The feed medium of claim 1, wherein a ratio of molar concentrations of N-acetylcysteine to L-cysteine is 0.1-0.4.

7. The feed medium of claim 1, having a pH of 5.0-9.0.

8. The feed medium of claim 1, having a pH of 6.5-7.6.

9. The feed medium of claim 1, wherein the feed medium is a serum-free medium and/or an animal protein-free medium.

10. The feed medium of claim 1, wherein the feed medium is a synthetic medium.

11. A powder medium comprising L-cysteine and N-acetylcysteine, for preparing a feed medium comprising 10-40 mM L-cysteine and 1-40 mM N-acetylcysteine.

12. A powder medium comprising L-cysteine and N-acetylcysteine, for preparing a feed medium having a ratio of molar concentrations of N-acetylcysteine to L-cysteine of 0.04-1.0.

13. The powder medium of claim 11, wherein the feed medium comprises 15-30 mM L-cysteine.

14. The powder medium of claim 11, wherein the feed medium comprises 2-10 mM N-acetylcysteine.

15. The powder medium of claim 11, wherein a ratio of molar concentrations of N-acetylcysteine to L-cysteine in the feed medium is 0.04-1.0.

16. The powder medium of claim 11, wherein a ratio of molar concentrations of N-acetylcysteine to L-cysteine in the feed medium is 0.1-0.4.

17. The powder medium of claim 11, wherein the feed medium has a pH of 5.0-9.0.

18. The powder medium of claim 11, wherein the feed medium has a pH of 6.5-7.6.

19. The powder medium of claim 11, wherein the feed medium is a serum-free medium and/or an animal protein-free medium.

20. The powder medium of claim 11, wherein the feed medium is a synthetic medium.

21. A method for culturing a cell, comprising adding the feed medium of any one of claims 1 to 10 to a medium for culturing the cell.

22. The method of claim 21, wherein the cell is an animal cell, a plant cell, a bacterial cell, or a fungal cell.

23. The method of claim 21, wherein the cell is an animal cell.

24. The method of claim 21, wherein the cell is a mammalian cell.

25. The method of claim 21, wherein the cell is a Chinese hamster ovary (CHO) cell.

26. The method of claim 21, wherein the cell expresses a recombinant protein.

27. The cell of claim 26, wherein the recombinant protein comprises an antibody or an antigen-binding fragment thereof.

28. The method of claim 27, wherein the antibody or the antigen-binding fragment thereof is derived from an IgG.

29. The method of claim 21, wherein heat load-induced precipitation of a medium component is absent.

30. A method for producing a recombinant protein, comprising adding the feed medium of any one of claims 1 to 10 to a medium for culturing a cell having a capability to produce a recombinant protein.

31. The method of claim 30, wherein the cell is an animal cell, a plant cell, a bacterial cell, or a fungal cell.

32. The method of claim 30, wherein the cell is an animal cell.

33. The method of claim 30, wherein the cell is a mammalian cell.

34. The method of claim 30, wherein the cell is a Chinese hamster ovary (CHO) cell.

35. The method of claim 30, wherein the recombinant protein comprises an antibody or an antigen-binding fragment thereof.

36. The method of claim 35, wherein the antibody or the antigen-binding fragment thereof is derived from an IgG.

37. The method of claim 30, wherein heat load-induced precipitation of a medium component is absent.

38. A method for improving stability of a liquid medium, comprising adding L-cysteine and/or N-acetylcysteine to a medium to achieve an L-cysteine concentration of 10-40 mM and an N-acetylcysteine concentration of 1-40 mM.

39. A method for improving stability of a liquid medium, comprising adding L-cysteine and/or N-acetylcysteine to a medium to achieve a ratio of molar concentrations of N-acetylcysteine to L-cysteine of 0.04-1.0.

40. A method for suppressing generation and/or precipitation of cystine in a liquid medium, comprising adding L-cysteine and/or N-acetylcysteine to a medium to achieve an L-cysteine concentration of 10-40 mM and an N-acetylcysteine concentration of 1-40 mM.

41. A method for suppressing generation and/or precipitation of cystine in a liquid medium, comprising adding L-cysteine and/or N-acetylcysteine to a medium to achieve a ratio of molar concentrations of N-acetylcysteine to L-cysteine of 0.04-1.0.

42. The method of any one of claims 38 to 41, comprising adding L-cysteine and N-acetylcysteine to the medium.

43. The method of any one of claims 38 to 41, wherein the liquid medium comprises 10-40 mM L-cysteine, and the method comprises adding N-acetylcysteine to the medium to achieve an N-acetylcysteine concentration of 1-40 mM.

44. The method of any one of claims 38 to 41, wherein the liquid medium comprises 1-40 mM N-acetylcysteine, and the method comprises adding L-cysteine to the medium to achieve an L-cysteine concentration of 10-40 mM.

45. The method of any one of claims 38 to 41, comprising adding L-cysteine and/or N-acetylcysteine to the medium to achieve an L-cysteine concentration of 15-30 mM.

46. The method of any one of claims 38 to 41, comprising adding L-cysteine and/or N-acetylcysteine to the medium to achieve an N-acetylcysteine concentration of 2-10 mM.

47. The method of claim 38 or 40, comprising adding L-cysteine and/or N-acetylcysteine to the medium to achieve a ratio of molar concentrations of N-acetylcysteine to L-cysteine of 0.04-1.0.

48. The method of any one of claims 38 to 41, comprising adding L-cysteine and/or N-acetylcysteine to the medium to achieve a ratio of molar concentrations of N-acetylcysteine to L-cysteine of 0.1-0.4.

49. The method of any one of claims 38 to 41, comprising adding L-cysteine and/or N-acetylcysteine to the medium to achieve a pH of 5.0-9.0 for the liquid medium.

50. The method of any one of claims 38 to 41, comprising adding L-cysteine and/or N-acetylcysteine to the medium to achieve a pH of 6.5-7.6 for the liquid medium.

51. An agent comprising L-cysteine and N-acetylcysteine, for improving stability of a liquid medium, wherein the liquid medium, after addition of the agent, comprises 10-40 mM L-cysteine and 1-40 mM N-acetylcysteine.

52. An agent comprising L-cysteine and N-acetylcysteine, for improving stability of a liquid medium, wherein a ratio of molar concentrations of N-acetylcysteine to L-cysteine in the liquid medium, after addition of the agent, is 0.04-1.0.

53. An agent comprising N-acetylcysteine, for improving stability of a liquid medium comprising 10-40 mM L-cysteine, wherein the liquid medium, after addition of the agent, comprises 1-40 mM N-acetylcysteine.

54. An agent comprising N-acetylcysteine, for improving stability of a liquid medium comprising L-cysteine, wherein a ratio of molar concentrations of N-acetylcysteine to L-cysteine in the liquid medium, after addition of the agent, is 0.04-1.0.

55. An agent comprising L-cysteine and N-acetylcysteine, for suppressing generation and/or precipitation of cystine in a liquid medium, wherein the liquid medium, after addition of the agent, comprises 10-40 mM L-cysteine and 1-40 mM N-acetylcysteine.

56. An agent comprising L-cysteine and N-acetylcysteine, for suppressing generation and/or precipitation of cystine in a liquid medium, wherein a ratio of molar concentrations of N-acetylcysteine to L-cysteine in the liquid medium, after addition of the agent, is 0.04-1.0.

57. An agent comprising N-acetylcysteine, for suppressing generation and/or precipitation of cystine in a liquid medium comprising 10-40 mM L-cysteine, wherein the liquid medium, after addition of the agent, comprises 1-40 mM N-acetylcysteine.

58. An agent comprising N-acetylcysteine, for suppressing generation and/or precipitation of cystine in a liquid medium comprising L-cysteine, wherein a ratio of molar concentrations of N-acetylcysteine to L-cysteine in the liquid medium, after addition of the agent, is 0.04-1.0.

59. The agent of any one of claims 51 to 58, wherein the liquid medium, after addition of the agent, comprises 15-30 mM L-cysteine.

60. The agent of any one of claims 51 to 58, wherein the liquid medium, after addition of the agent, comprises 2-10 mM N-acetylcysteine.

61. The agent of any one of claims 51, 53, 55, and 57, wherein a ratio of molar concentrations of N-acetylcysteine to L-cysteine in the liquid medium, after addition of the agent, is 0.04-1.0.

62. The agent of any one of claims 51 to 58, wherein a ratio of molar concentrations of N-acetylcysteine to L-cysteine in the liquid medium, after addition of the agent, is 0.1-0.4.

63. The agent of any one of claims 51 to 58, wherein the liquid medium, after addition of the agent, has a pH of 5.0-9.0.

64. The agent of any one of claims 51 to 58, wherein the liquid medium, after addition of the agent, has a pH of 6.5-7.6.

65. A media additive comprising N-acetylcysteine and L-cysteine, for preparing the feed medium of any one of claims 1 to 10 or the powder medium of any one of claims 11 to 20.

66. A media additive comprising N-acetylcysteine, for preparing the feed medium of any one of claims 1 to 10 or the powder medium of any one of claims 11 to 20.
